# EUROPÄISCHE PATENTANMELDUNG

(11) **EP 3 686 177 A1**
(43) Veröffentlichungstag der Anmeldung: **29.07.2020**
(21) Anmeldenummer: 19154007.9
(22) Anmeldetag: 28.01.2019
(51) Int. Cl.: C07C 7/17, C07C 7/148, C07C 11/107, C07C 2/06

(54) **VERFAHREN UND ANLAGE ZUR HERSTELLUNG VON ALPHA-OLEFINEN**

(71) Anmelder: Linde GmbH, 82049 Pullach (DE)
(72) Erfinder: Winkler, Florian, 81241 München (DE); Schneider, Richard, 82449 Uffing am Staffelsee (DE); Mündl, Florian, 83624 Otterfing (DE)
(74) Vertreter: Frommberger, Moritz

(57) **Zusammenfassung**

Es wird ein Verfahren (100) zur Herstellung von linearen Alpha-Olefinen vorgeschlagen, bei dem Ethylen in einem Einsatzgemisch unter Erhalt eines Produktgemischs, das Alpha-Olefine mit unterschiedlicher Kettenlänge und Nebenverbindungen enthält, einer katalytischen Oligomerisierung (1) unterworfen wird. In einer Primärfraktionierung (2) wird unter Verwendung zumindest eines Teils des Produktgemischs eine Primärfraktion gebildet, und in einer Sekundärfraktionierung (4) wird unter Verwendung zumindest eines Teils der Primärfraktion eine Sekundärfraktion gebildet. Die Primärfraktionierung (2) und die Sekundärfraktionierung (4) werden derart durchgeführt, dass die Primärfraktion und die Sekundärfraktion überwiegend eines der Alpha-Olefine enthalten und arm an oder frei von anderen Alpha-Olefinen sind, dass die Primärfraktion eine oder mehrere der Nebenverbindungen enthält, und dass die Sekundärfraktion gegenüber der Primärfraktion an der einen oder an den mehreren Nebenverbindungen abgereichert ist. In einem Zwischenschritt (3) zwischen der Primärfraktionierung (2) und der Sekundärfraktionierung (4), welchem zumindest ein Teil der Primärfraktion unterworfen wird, werden die eine oder die mehreren Nebenverbindungen zumindest zu einem Teil zu einer oder zu mehreren Folgeverbindungen umgesetzt und die eine oder die mehreren Folgeverbindungen werden in der Sekundärfraktionierung (4) zumindest zum Teil abgetrennt. Der Zwischenschritt (3) wird derart durchgeführt wird, dass nicht mehr als 0,8% des in der Primärfraktion oder deren dem Zwischenschritt unterworfenem Teil überwiegend enthaltenen Alpha-Olefins umgesetzt werden.

## Beschreibung

Die Erfindung betrifft ein Verfahren zur Herstellung eines Alpha-Olefins und eine entsprechende Anlage gemäß den Oberbegriffen der unabhängigen Patentansprüche.

### Stand der Technik

Die Herstellung von Alpha-Olefinen (α-Olefinen) durch Oligomerisierung von Ethylen ist bekannt und in der Fachliteratur, beispielsweise bei D. Steinborn, Fundamentals of Organometallic Catalysis, Wiley-VCH, 2012, insbesondere Kapitel 8, "Oligomerization of Olefins", beschrieben. Wichtige Verfahrensvarianten sind beispielsweise der Shell Higher Olefin Process (SHOP) und der Alpha-SABLIN-Prozess, wie bei Steinborn in den Unterkapiteln 8.4.1 und 8.4.2 und anderenorts erläutert.

In den genannten Verfahren werden Gemische aus geradzahligen Alpha-Olefinen in einer typischen Schulz-Flory-Verteilung gebildet, wobei der Schwerpunkt typischerweise auf der Bildung 1-Buten und 1-Hexen liegt.

Die genannten Oligomerisierungsprozesse und im Speziellen der Alpha-SABLIN-Prozess ist in der Produktverteilung ausgesprochen flexibel. So können Verteilungen mit Ausbeuten von beispielsweise 30 bis 40 Gewichtsprozent an 1-Buten und 1-Hexen eingestellt werden. In diesem Fall erfüllen i.d.R. die jeweils gebildeten Fraktionen die Produktqualitäten die Anforderungen des Marktes bezüglich Fremdverbindungen.

Bei der Einstellung höherer Ausbeuten von beispielsweise 40 bis 70 Gewichtsprozent an 1-Buten und 1-Hexen werden hingegen die Anforderungen des Marktes an die Fraktionen aufgrund einer erhöhten Produktion von verzweigten Olefinen in den einzelnen Fraktionen nicht mehr erfüllt. Gleichwohl sind entsprechend hohe Ausbeuten erwünscht. Daher muss in solchen Fällen insbesondere eine sogenannte Superfraktionierung der 1-Buten-, der 1-Hexen-, der 1-Octen- und/oder der 1-Decen-Fraktion und eine Abtrennung verzweigter Olefine erfolgen.

Beispielsweise kann die 1-Hexen-Fraktion in den genannten Fällen je nach der eingestellten Verteilung mit 0,5 bis 2 Gewichtsprozent der etwas schwerer siedenden Komponente 2-Ethyl-1-Buten verunreinigt sein.

Die 1-Octen- und die 1-Decen-Fraktion sind typischerweise jeweils durch leichter siedene Komponenten verunreinigt, die jedoch sehr nahe an den jeweiligen Alpha-Olefinen sieden, nämlich durch 2-Ethyl-1-Hexen und 2-Ethyl-1-Octen.

Die vorliegende Erfindung stellt sich vor diesem Hintergrund insbesondere die Aufgabe, verbesserte Möglichkeiten zur Herstellung reiner bzw. spezifikationsgerechter Alpha-Olefinfraktionen zu schaffen.

### Offenbarung der Erfindung

Vor diesem Hintergrund werden ein Verfahren zur Herstellung eines von Alpha-Olefinen und eine entsprechende Anlage gemäß den Oberbegriffen der unabhängigen Patentansprüche vorgeschlagen. Ausgestaltungen sind Gegenstand der abhängigen Patentansprüche und der nachfolgenden Beschreibung.

Vor der Erläuterung der erfindungsgemäßen Merkmale werden weitere Grundlagen der Erfindung erläutert und verwendete Begriffe definiert.

Generell wird hier unter einer "Primärfraktionierung" eines Gemischs von Alpha-Olefinen die Bildung eines Komponentengemischs verstanden, das ein lineares Alpha-Olefin einer bestimmten Kohlenstoffzahl enthält, aber arm an oder frei von linearen Alpha-Olefinen anderer Kohlenstoffzahlen ist. Ein derartiges Komponentengemisch wird nachfolgend auch als "Primärfraktion" eines entsprechenden linearen Alpha-Olefins, beispielsweise als 1-Buten-, 1-Hexen-, 1-Octen- oder 1-Decen-Primärfraktion bezeichnet. Das jeweils kenntzeichnende, da im überwiegenden Anteil enthaltene, lineare Alpha-Olefin wird nachfolgend jeweils auch als die "Hauptkomponente" in einer entsprechenden Primärfraktion bezeichnet.

Eine entsprechende Primärfraktion kann zusätzlich zu der jeweiligen Hauptkomponente leichter siedende und/oder schwerer siedende Komponenten aufweisen. Dies ist in dem erfindungsgemäß vorgeschlagenen Verfahren der Fall. Definitionsgemäß handelt es sich hierbei aber nicht aber andere lineare Alpha-Olefine, sondern beispielsweise um verzweigte Olefine, Paraffine oder lineare Olefine mit einer nicht endständigen Doppelbindung. Diese besitzen jeweils einen Siedepunkt, der einen Abstand zu einem Siedepunkt der Hauptkomponente aufweist, wobei dieser Abstand geringer ist als ein Abstand zwischen dem Siedepunkt der Hauptkomponente und eines linearen Alpha-Olefins, das zwei Kohlenstoffe mehr oder weniger als die Hauptkomponente aufweist.

Die erwähnten leichter und/oder schwerer siedenden Komponenten werden in einer sogenannten Superfraktionierung, nachfolgend auch als "Sekundärfraktionierung" bezeichnet, abgetrennt. Die in der Sekundärfraktionierung gebildeten Fraktionen, die nachfolgend auch als "Sekundärfraktionen" bezeichnet werden, sind damit gegenüber den entsprechenden Primärfraktionen an den leichter und/oder schwerer siedenden Komponenten abgereichert, jedoch aus technischen Gründen typischerweise nicht völlig frei von diesen. In einer 1-Hexen-Sekundärfraktion sind typischerweise nicht mehr als 0,5 Gewichtsprozent, in einer 1-Octen-Sekundärfraktion typischerweise nicht mehr als 3 Gewichtsprozent und in einer 1-Decen-Sekundärfraktion typischerweise nicht mehr als 3,5 bis 4 Gewichtsprozent an schwerer siedenden Komponenten enthalten. In einer jeweiligen Primärfraktion können entsprechende leichter und/oder schwerer siedende Komponenten hingegen deutlich höheren Gehalten auftreten.

Durch die Superfraktionierung entstehen zusätzliche Verluste sowie erhöhte operative Kosten sowie erhöhte Investitionskosten. Dies gilt umso mehr, je näher die Siedepunkte dieser leichteren und/oder schwereren Komponenten an jenen der jeweiligen Hauptkomponente liegen, da die Abtrennung hierdurch erschwert wird. In besonderer Weise ist hierbei die 1-Hexen-Fraktion betroffen, weil in dieser die sehr nahe siedende Komponente 2-Ethyl-1-Buten den einzustellenden Restgehalten entspricht oder diese übetrifft.

### Vorteile der Erfindung

Aus der RU 2 206 557 C1 ist ein Verfahren bekannt, bei 2-Ethyl-1-buten in einem Komponentengemisch mit 1-Hexen durch selektive Isomerisierung von zu 3-Methyl-2-Penten an einem Katalysator umgesetzt wird. Der Katalysator ist ein makroporöses Sulfokationit mit einer volumenbezogenen Kapazität von 3,5 bis 4,5 mg × equ. H⁺/g. Das Verfahren wird bei einer Bettgeschwindigkeit von 1 bis 10 h⁻¹ und bei einer Temperatur von 40 bis 80 °C durchgeführt. Der Gehalt von 2-Ethyl-1-Buten in dem Komponentengemisch liegt bei mehr als 1 Gew.-%, wobei in einem konkreten Beispiel 4,3 Gew.-% genannt sind. Der Gehalt an 1-Hexen liegt bei mehr als 95 Gew.-%. Das enthaltene 2-Ethyl-1-Buten wird zu 86 bis 97% umgesetzt, wobei sich Konversionsverluste von 1 bis 2,6% an 1-Hexen ergeben.

Während unter den hier relevanten Bedingungen 1-Hexen einen Siedepunkt von 63 bis 64 °C und 2-Ethyl-1-Buten einen Siedepunkt von 64 b is 65 °C aufweist, und diese Komponenten daher nur sehr aufwendig oder gar nicht voneinander trennbar sind, besitzt cis/trans-3-Methyl-2-Penten einen Siedepunkt von 67 bis 72 °C und ist daher deutlich einfacher abtrennbar. Durch einen entsprechenden Zwischenschritt erleichtert sich daher die Superfraktionierung bzw. Sekundärfraktionierung deutlich.

Erfindungsgemäß wurde nun überraschend erkannt, dass sich ein derartiger Zwischenschritt auch mit einer deutlich geringeren Umsetzung von 1-Hexen bzw. eines anderen Alpha-Olefins durchgeführt werden kann. Auf diese Weise kann ein Verfahren geschaffen werden, bei dem die Produkverluste deutlich reduziert sind. Bei der unerwünschetn Umsetzung von 1-Hexen handelt es sich um eine Nebenreaktion, bei der cis/trans-2-Hexen und/oder cis/trans-3-Hexen gebildet werden.

Maßnahmen zur Reduzierung der unerwünschten Umsetzung umfassen dabei im Rahmen der vorliegenden Erfindung insbesondere die Verwendung eines Reaktionsmoderators, beispielsweise Wasser, und/oder die Verwendung eines weniger sauren Katalysators wie unten erläutert.

Insgesamt schlägt die Erfindung ein Verfahren zur Herstellung von linearen Alpha-Olefinen vor, bei dem Ethylen in einem Einsatzgemisch unter Erhalt eines Produktgemischs, das Alpha-Olefine mit unterschiedlicher Kettenlänge und Nebenverbindungen enthält, einer katalytischen Oligomerisierung unterworfen wird.

Im Rahmen der vorliegenden Erfindung können dabei grundsätzlich sämtliche Verfahren zur katalytischen Oligomerisierung von Ethylen eingesetzt werden, die aus dem Stand der Technik bekannt sind. Auf die obigen Erläuterungen wird daher verwiesen. Insbesondere kann die katalytische Oligomerisierung in Form des bekannten SABLIN-Verfahrens erfolgen, wie ebenfalls eingangs erläutert. Die katalytische Oligomerisierung kann insbesondere mit einer Ausbeute von 40 bis 70 Gewichtsprozent an 1-Buten und 1-Hexen durchgeführt werden.

Im Rahmen der Erfindung können insbesondere 1-Buten, 1-Hexen, 1-Octen und/oder 1-Decen als lineare Alpha-Olefine gebildet werden. Bei den Nebenverbindungen kann es sich insbesondere um 2-Ethyl-1-Buten und 2-Ethyl-1-Hexen handeln bzw. allgemeiner um 2-Ethyl-1-Olefine mit der gleichen Kohlenstoffzahl wie die jeweiligen lineare Alpha-Olefine, die als Zielprodukte gebildet werden. Weitere im Rahmen eines entsprechenden Verfahrens möglicherweise gebildete Nebenverbindungen umfassen cis/trans-3-Hexen, n-Hexan, cis/trans-2-Hexen, cis/trans-3-Octen, cis/trans-4-Octen, trans-2-Octen, n-Octan und cis-2-Octen. Bei den Nebenverbindungen handelt es sich nicht um lineare Alpha-Olefine.

In einer Primärfraktionierung wird im Rahmen der vorliegenden Erfindung unter Verwendung zumindest eines Teils des Produktgemischs aus der katalytischen Oligomerisierung eine Primärfraktion gebildet, und in einer Sekundärfraktionierung wird unter Verwendung zumindest eines Teils der Primärfraktion eine Sekundärfraktion gebildet. Zu den Begriffen der Primärfraktion und der Sekundärfraktion bzw. entsprechenden Fraktionierungsschritten sei auf die obigen Erläuterungen verwiesen. Die Sekundärfraktionierung kann insbesondere in Form einer bekannten Superfraktionierung durchgeführt werden. Primär- und Sekundärfraktionierung können insbesondere in Form thermischer Trennschritte, insbesondere in Form von Rektifikationen, durchgeführt werden.

Gemäß einer besonders bevorzugten Ausgestaltung der vorliegenden Erfindung kann die Primärfraktion getrocknet werden, bevor sie der Sekundärfraktionierung unterworfen wird. Zur Trocknung können sämtliche bekannten Verfahren zum Einsatz kommen, beispielsweise eine adsorptive Trocknung unter Verwendung von geeigneten Adsorptionsmaterialien. Ist im Rahmen der vorliegenden Erfindung ein bestimmter Wassergehalt in einem Verfahrensschritt vorteilhaft, kann eine Trocknung auch erst stromab dieses Verfahrensschritts erfolgen oder vollständig unterbleiben.

Im Rahmen der vorliegenden Erfindung werden die Primärfraktionierung und die Sekundärfraktionierung derart durchgeführt, dass die Primärfraktion und die Sekundärfraktion überwiegend eines der linearen Alpha-Olefine enthalten und arm an oder frei von anderen Alpha-Olefinen sind, dass die Primärfraktion eine oder mehrere der Nebenverbindungen enthält, und dass die Sekundärfraktion gegenüber der Primärfraktion an der einen oder an den mehreren Nebenverbindungen abgereichert oder (soweit technisch sinnvoll und möglich) frei hiervon ist. Mit anderen Worten wird jeweils sichergestellt, dass die Primärfraktion neben der jeweiligen Hauptkomponente in Form des linearen Alpha-Olefins vorzugsweise keine weiteren linearen Alpha-Olefine enthält bzw. diese nur in sehr geringem Umfang enthalten sind.

Ist hier davon die Rede, dass "eine" Primärfraktion gebildet wird, schließt dies selbstverständlich die Bildung weiterer Primärfraktionen mit entsprechenden anderen linearen Alpha-Olefinen als Hauptkomponente nicht aus. Beispielsweise können als Primärfraktionen eine 1-Hexen-Primärfraktion, eine 1-Octen-Primärfraktion, eine 1-Decen-Primärfraktion und/oder eine 1-Dodecen-Primärfraktion gebildet werden, die jeweils entsprechende Nebenverbindungen enthalten.

Erfindungsgemäß werden in einem Zwischenschritt zwischen der Primärfraktionierung und der Sekundärfraktionierung, welchem zumindest ein Teil der Primärfraktion unterworfen wird, die eine oder die mehreren Nebenverbindungen zumindest zu einem Teil zu einer oder zu mehreren Folgeverbindungen umgesetzt, und die eine oder die mehreren Folgeverbindungen werden anschließend in der Sekundärfraktionierung zumindest zum Teil abgetrennt.

Als Nebenverbindung in einer 1-Hexen-Primärfraktion kann, wie erläutert, insbesondere 2-Ethyl-1-buten enthalten sein, welches sich, wie erwähnt, in seinem Siedepunkt nur gering von 1-Hexen unterscheidet. Durch die Umsetzung in dem Zwischenschritt kann hieraus cis/trans-3-Methyl-2-penten gebildet werden, das mit seinem höheren Siedepunkt deutlich einfacher abgetrennt werden kann.

In einer 1-Octen-Primärfraktion kann beispielsweise 2-Ethyl-1-Hexen als Nebenverbindung vorliegen. Der Siedepunkt von 1-Octen liegt bei 121 °C, jener von 2-Ethyl-1-Hexen bei 120 °C. Durch die Umsetzung von 2 -Ethyl-1-Hexen zu cis/trans-3-Methyl-2-Hepten mit einem Siedepunkt von 122 bis 126 °C wird die Abtrennung auch dieser Nebenverbindung erleichtert. Weil diese Nebenverbindung durch die erwähnte Umsetzung auf die Siedelage anderer Octenisomere verschoben wird, kann es gemeinsam mit diesen abgetrennt werden. So sieden 2-Octen und 3-Octen ebenfalls oberhalb von 122°C. Entsprechendes gilt für andere Nebenverbindungen in gleicher oder vergleichbarer Weise.

Die Erfindung zeichnet sich nun dadurch aus, dass der Zwischenschritt derart durchgeführt wird, dass nicht mehr als 0,8%, insbesondere nicht mehr als 0,5% oder 0,2% bezogen auf Gewichts-, Volumen- oder Molbasis des Alpha-Olefins, das in der Primärfraktion oder in dem Teil hiervon, der dem Zwischenschritt unterworfen wird, überwiegend enthalten ist, umgesetzt werden.

Insbesondere kann bzw. können im Rahmen der vorliegenden Erfindung die eine oder die mehreren Nebenverbindungen in dem Zwischenschritt bis auf einen Restgehalt von höchstens 0,4%, insbesondere von höchstens 0,2% oder 0,1% auf Gewichts-, Mol- oder Volumenbasis umgesetzt werden. Dieser Restgehalt kann sich beispielsweise auf ein 2-Ethyl-1-Olefin mit der gleichen Kohlenstoffzahl wie das in der Primär- und der Sekundärfraktion überwiegend enthaltene lineare Alpha-Olefin beziehen und bezeichnet insbesondere den Gehalt in der Primärfraktion nach der Umsetzung in dem Zwischenschritt. Die Umsetzung des 2-Ethyl-1-Olefins in dem Zwischenschritt kann insbesondere zu einem cis/trans-3-Methyl-2-Olefin mit wiederum der gleichen Kohlenstoffanzahl erfolgen.

Mit anderen Worten erfolgt im Rahmen der vorliegenden Erfindung vorteilhafterweise eine möglichst vollständige Umsetzung des 2-Ethyl-Olefins mit der gleichen Kohlenstoffzahl, also je nach Fraktion des 2-Ethyl-1-Butens, des 2-Ethyl-1-Hexens oder des Ethyl-1-Octens. Die Umsetzung erfolgt insbesondere auf einen Restgehalt im bereits oben angegebenen Bereich.

Wie erwähnt, können Maßnahmen zur Reduzierung der unerwünschten Umsetzung des jeweiligen linearen Alpha-Olefins dabei insbesondere die Verwendung eines Reaktionsmoderators, beispielsweise Wasser, und/oder die Verwendung eines weniger sauren Katalysators wie unten erläutert umfassen.

In einer ersten Ausgestaltung des erfindungsgemäßen Verfahrens wird der Zwischenschritt in Anwesenheit eines Reaktionsmoderators durchgeführt, bei dem es sich insbesondere um Wasser handeln kann. Dies gilt insbesondere für eine 1-Hexen-Primärfraktion. Wasser kann im Rahmen der vorliegenden Erfindung insbesondere in einem Gehalt von 20 oder 30 Gew.-ppm bis 150 Gew.-ppm oder 200 Gew.-ppm, insbesondere von 50 Gew.-ppm bis 100 Gew.-ppm, verwendet werden. Wasser kann bereits in dem der Primärfraktionierung unterworfenen Produktgemisch enthalten sein oder separat zugegeben werden. Diese Verfahrensvariante hat den besonderen Vorteil, dass die Säurestärke des Katalysators gezielt gemindert werden kann, so dass dadurch die unerwünschte Isomerisierungen des alpha Olefins stark reduziert werden kann. Die gewünschte Isomerisierung der Zielkomponente bleibt jedoch in ausreichender Weise erhalten. Insbesondere für höherkettige alpha-Olefine kann der Moderator entfallen.

Das Verfahren kann gemäß dieser ersten Ausgestaltung der Erfindung insbesondere unter Verwendung eines stark sauren lonenaustauscherharzes in dem Zwischenschritt durchgeführt werden. Als das stark saure lonenaustauscherharz kann insbesondere ein makroporöses Sulfokationit verwendet werden, beispielsweise ein kommerziell verfügbares makroporöses Sulfokationit.

Das stark saure lonenaustauscherharz kann in der ersten Ausgestaltung der vorliegenden Erfindung insbesondere eine volumenbezogene Kapazität von mindestens 4 eq/kg aufweisen, und/oder der Zwischenschritt kann hierbei bei einer Bettgeschwindigkeit von 5 bis 40 h⁻¹ bei Temperaturen von 30 bis 60 °C, insbesondere von 40 bis 50 °C, durchgeführt werden. Unter diesen Reaktionsbedingungen ergeben sich die Vorteile, die durch die Verwendung des Reaktionsmoderators erzielbar sind, in besonderer Weise.

In der ersten Ausgestaltung der vorliegenden Erfindung ergibt sich eine moderate Umsetzung von insbesondere nur 0,1 bis 0,5 oder 0,8% des in der Primärfraktion oder deren dem Zwischenschritt unterworfenem Teil überwiegend enthaltenen Alpha-Olefins bei Umsetzungen der zu isomierenden Nebenverbindung von 85 bis 95%. Das Verfahren gemäß der ersten Ausgestaltung der vorliegenden Erfindung erhöht daher die Ausbeute gegenüber dem Stand der Technik beträchtlich.

In einer zweiten Ausgestaltung der vorliegenden Erfindung wird der Zwischenschritt unter Verwendung eines aluminiumoxidbasierten Katalysators durchgeführt, der insbesondere Chi- und Gamma-Dialuminiumtrioxid aufweist. Diese Verfahrensvariante hat den besonderen Vorteil, dass die Säurestärke bereits in einem Bereich liegt, in dem kein Moderator erforderlich ist und somit eine geringe Konversion des Alpha-Olfins gegenüber jener des 2-Ethyl-1-Olefins erreicht wird.

Der aluminiumoxidbasierte Katalysator kann in der zweiten Ausgestaltung der vorliegenden Erfindung insbesondere eine Oberfläche von 450 bis 460 m²/g aufweisen, und der Zwischenschritt kann hierbei unter Verwendung einer Bettgeschwindigkeit von 1 bis 12 h⁻¹ durchgeführt werden. Unter diesen Reaktionsbedingungen ergeben sich die Vorteile der zweiten Ausgestaltung in besonderer Weise.

In der zweiten Ausgestaltung der vorliegenden Erfindung ergibt sich eine besonders geringe Umsetzung von insbesondere weniger als 0,1% des in der Primärfraktion oder deren dem Zwischenschritt unterworfenem Teil überwiegend enthaltenen Alpha-Olefins bei einer Umsetzung der zu isomierenden Nebenverbindung von 85 bis 95% und damit eine nochmals verbesserte Ausbeuteerhöhung.

In beiden zuvor erläuterten Ausgestaltungen der vorliegenden Erfindung, d.h. der ersten und der zweiten Ausgestaltung, hat sich als besonders vorteilhaft erwiesen, den Zwischenschritt auf einem Temperaturniveau von 60 bis 100 °C, insbesondere von 70 bis 90 °C, und auf einem Druckniveau von 1,0 bis 4, 0 bar Absolutdruck durchzuführen. In sämtlichen Ausgestaltungen der vorliegenden Erfindung kann ferner eine Regenerierung des verwendeten Katalysators vorgenommen werden, wobei beispielsweise auch mehrere Reaktoren im Wechselbetrieb verwendet werden können, von denen jeweils zumindest einer für den Zwischenschritt zur Verfügung steht.

### Beispiele

Das in der erwähnten RU 2 206 557 C1 offenbarte Verfahren ("Referenz") wurde vorliegend mit drei Beispielen ("Beispiel 1" bis "Beispiel 3") verglichen. Die Ergebnisse dieses Vergleichs sowie die jeweils verwendeten Katalysatoren, weitere Reaktionsbedingungen sind in Tabelle 1 zusammengefasst.

In der Referenz und in den Beispielen 1 und 2 wurde ein makroporöses Sulfokationit als Katalysator verwendet, wobei in den Beispielen 1 und 2, wie in der Referenz, jeweils ein kommerziell erhältliches Produkt eingesetzt wurde. Die Beispiele 1 und 2 unterscheiden sich voneinander im Wesentlichen durch die Menge des als Reaktionsmoderator eingesetzten Wassers.

In Beispiel 3 wurde im Gegensatz dazu ein amorphes Chi- und Gamma-Dialuminiumtrioxid als Katalysator verwendet, wobei ebenfalls auf ein kommerziell erhältliches Produkt zurückgegriffen wurde. Wie in Beispiel 1 wurde jedoch eine ausgesprochen geringe Wassermenge verwendet.

Wie sich aus dem Vergleich der Ergebnisse ergibt, können dabei in Beispiel 1, bei dem ein vergleichbarer Katalysator wie in der Referenz und eine geringe Wassermenge als Reaktionsmoderator verwendet wurden, keine nennenswerten Verbesserungen hinsichtlich der Umsetzung von 1-Hexen gegenüber der Referenz erzielt werden. Deutliche Verbesserungen treten jedoch bei der Verwendung höherer Wassermengen gemäß Beispiel 2 und bei der Verwendung des Chi- und Gamma-Dialuminiumtrioxids als Katalysator gemäß Beispiel 3 auf.

**Tabelle 1**

| **Parameter** | **Referenz** | **Beispiel 1** | **Beispiel 2** | **Beispiel 3** |
|---|---|---|---|---|
| Bettgeschwindigkeit | 1-10 h⁻¹ | 30-90 h⁻¹ | 5-40 h⁻¹ | 1-10 h⁻¹ |
| Temperatur | 40-80 °C | 40-60 °C | 40-60 °C | 60-90 °C |
| Absolutdruck | 1,0-3,0 bar | 1,0-4,0 bar | 1,0-4,0 bar | 1,0-4,0 bar |
| Katalysator | Amberlyst 15 | Lewatit K-2649 | Lewatit K-2649 | Selexsorb CDX |
| Typ Katalysator | makroporöses Sulfokationit amorphes Chi- und Gamma-Dialuminiumtrioxid | | | amorphes Chi- und Gamma-Dialuminiumtrioxid |
| Eigenschaften Katalysator | volumenbezogene Kapazität 3,5-4,5 mg × equ. H⁺/g | volumenbezogene Kapazität min. 4,7 eq/kg (trocken) | | Oberfläche 450-460 m²/g |
| Alpha-Olefin im Reaktionseinsatz | 95 Gew.-% | 97 bis 99 Gew.-% | | |
| 2-Ethyl-1-Buten im Reaktionseinsatz | 4,3 Gew.-% | 1 bis 1,5 Gew.-% | | |
| Wassergehalt | - | < 20 Gew.-ppm | < 150 Gew.-ppm | < 20 Gew.-ppm |
| Umsatz alpha Olefin | 1 bis 2,6% | 0,5 bis 2% | 0,1 bis 0,5% | < 0,1% |
| Umsatz 2-Ethyl-1-Buten | 86 bis 97% | 85 bis 95% | | |

In einem weiteren Beispiel (Beispiel 4) wurde ebenso ein amorphes Chi- und Gamma-Dialuminiumtrioxid als Katalysator verwendet, wobei ebenfalls auf ein kommerziell erhältliches Produkt zurückgegriffen wurde. Es wurde gezeigt, dass die Deaktivierung des Katalysators durch den Wassergehalt im Reaktionseinsatz verursacht wird und zudem reversibel ist. Durch gängige Methoden der Regenerierung kann die Isomerisierungseigenschaft vollständig auf die Anfangsaktivität wiederhergestellt werden. Die Frequenz der Regenerierung kann vermieden und/oder reduziert werden, indem man einen Vortrockner vorschaltet. In Beispiel 4 wurde mit einer Bettgeschwindigkeit von 12 h⁻¹ bei einer Temperatur von 90 °C, einem Druck von 4 bar oberhalb des Atmoshärendrucks, mit 3,8 g Katalysator und mit einer Feuchte im Reaktionseinatz von 40 Gew.-ppm gearbeitet. Der Vortrockner wurde mit 7 g Selexsorb CDX bei ca. 20°C betrieben. Die Ergebnisse sind in Tabelle 2 zusammengefasst. In dieser Tabelle sind in den Spalten 2 und 3 der Gehalt an 2-Ethyl-1-Buten und 1-Hexen im Reaktionseinsatz angegeben.

**Tabelle 2**

| Zeit | Gehalt 2-Ethyl-1-Buten | Gehalt 1-Hexen | Umsetzung 1-Hexen | Umsetzung 2-Ethyl-1-Buten |
|---|---|---|---|---|
| 0 h | 1,0 Gew.-% | 97,36 Gew.-% | | |
| 2 h | 0,15 Gew.-% | 97,33 Gew.-% | <0,1% | 85% |
| 24 h | 0,28 Gew.-% | 97,33 Gew.-% | <0,1% | 72% |

| Regenerierung | | | | |
|---|---|---|---|---|
| 30 h | 0,15 Gew.-% | 97,32 Gew.-% | <0,1% | 85% |

| Einbau Vortrockner (7g Selexsorb CDX) | | | | |
|---|---|---|---|---|
| 32 h | 0,15 Gew.-% | 97,32 | <0,1% | 85% |
| 56 h | 0,14 Gew.-% | 97,29 | <0,1% | 86% |

In einem weiteren Beispiel (Beispiel 5) wurde ebenso ein amorphes Chi- und Gamma-Dialuminiumtrioxid als Katalysator verwendet, wobei ebenfalls auf ein kommerziell erhältliches Produkt zurückgegriffen wurde. Es wurde gezeigt, dass die Verluste an 1-Hexen Verluste über die Bettgeschwindigkeit minimiert werden können. Es wurden eine Temperatur von 90°C, ein Druck von 4bar oberha Ib des Atmosphärendrucks und 3,8 g Katalysator verwendet. Die Feuchte im Reaktionseinsatz lag bei weniger als 20 Gew.-ppm). Die Ergebnisse sind in nachstehender Tabelle 3 angegeben.

**Tabelle 3**

| Bettgeschwindigkeit | Gehalt 2-Ethyl-1-Buten | Gehalt 1-Hexen | Umsetzung 1-Hexen | Umsetzung 2-Ethyl-1-Buten |
|---|---|---|---|---|
| 0 h⁻¹ | 1,07 Gew.-% | 97,90 Gew.-% | | |
| 3 h⁻¹ | 0,05 Gew.-% | 97,82 Gew.-% | 0,08% | 95% |
| 6 h⁻¹ | 0,07 Gew.-% | 97,83 Gew.-% | 0,07% | 93% |
| 12 h⁻¹ | 0,19 Gew.-% | 97,88 Gew.-% | 0,02% | 82% |

### Zeichnungen

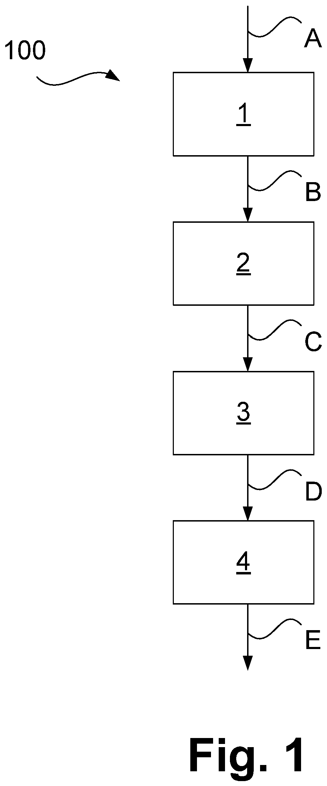

In Figur 1 ist ein Verfahren zur Herstellung von linearen Alpha-Olefinen gemäß einer Ausführungsform der Erfindung in Form eines schematischen Ablaufplans dargestellt und insgesamt mit 100 bezeichnet.

In dem Verfahren 100 wird Ethylen in einem Einsatzgemisch A unter Erhalt eines Produktgemischs B, das Alpha-Olefine mit unterschiedlicher Kettenlänge und Nebenverbindungen enthält, einer katalytischen Oligomerisierung 1 unterworfen.

In einer Primärfraktionierung 2 wird unter Verwendung zumindest eines Teils des Produktgemischs B eine Primärfraktion C gebildet, und in einer Sekundärfraktionierung 4 wird unter Verwendung zumindest eines Teils der Primärfraktion C eine Sekundärfraktion gebildet.

Die Primärfraktionierung 2 und die Sekundärfraktionierung 4 werden derart durchgeführt, dass die Primärfraktion und die Sekundärfraktion überwiegend eines der Alpha-Olefine enthalten und arm an oder frei von anderen Alpha-Olefinen sind, dass die Primärfraktion eine oder mehrere der Nebenverbindungen enthält, und dass die Sekundärfraktion gegenüber der Primärfraktion an der einen oder an den mehreren Nebenverbindungen abgereichert ist.

In einem Zwischenschritt 3 zwischen der Primärfraktionierung 2 und der Sekundärfraktionierung 4, welchem zumindest ein Teil der Primärfraktion C unterworfen wird, werden die eine oder die mehreren Nebenverbindungen zumindest zu einem Teil zu einer oder zu mehreren Folgeverbindungen umgesetzt.

Die eine oder die mehreren, in dem Zwischenschritt 3 gebildeten Folgeverbindungen werden in der Sekundärfraktionierung 4 zumindest zum Teil abgetrennt. Der Zwischenschritt 3 wird derart durchgeführt wird, dass nicht mehr als 0,8% des in der Primärfraktion oder deren dem Zwischenschritt unterworfenem Teil überwiegend enthaltenen Alpha-Olefins umgesetzt werden.

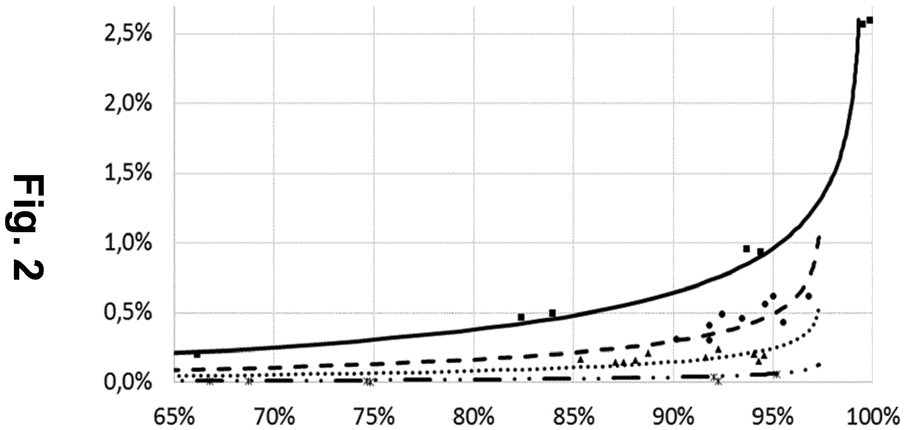

In Figur 2 sind die Ergebnisse einer Versuchsreihe dargestellt, die mit Lewatit K-2649 als Katalysator (also wie in den obigen Beispielen 2 und 3 einem makroporösen Sulfokationit) und mit unterschiedlichen Wassermengen als Moderator durchgeführt wurden. In dem Diagramm der Figur 2 sind die (erwünschte) Umsetzung von 2-Ethyl-1-buten auf der Abszisse und die (unerwünschte) Umsetzung von 1-Hexen auf der Ordinate jeweils in Prozent angegeben.

Die durchgezogene Linie mit quadratischen Datenpunkten veranschaulicht die erzielten Werte ohne Wasser. Die gestrichelte Linie mit runden Datenpunkten veranschaulicht die erzielten Werte bei einer Wassermenge von 60 Gew.-ppm. Die punktierte Linie mit dreieckigen Datenpunkten veranschaulicht die erzielten Werte bei einer Wassermenge von 100 Gew.-ppm. Die strichpunktierte Linie mit sternförmigen Datenpunkten veranschaulicht die erzielten Werte bei einer Wassermenge von 60 Gew.-ppm.

Aus den in Figur 2 veranschaulichten Werten geht eindeutig hervor, das eine rein trockene Anwendung zu hohen Verlusten von 1-Hexen führt. Eine Anwendung mit ca. 100 bis 130 Gew.-ppm Wasser am Reaktoraustritt führt zu erheblichen Verbesserungen bezüglich dieser Verluste.

## Patentansprüche

1. Verfahren (100) zur Herstellung von linearen Alpha-Olefinen, bei dem
- Ethylen in einem Einsatzgemisch unter Erhalt eines Produktgemischs, das Alpha-Olefine mit unterschiedlicher Kettenlänge und Nebenverbindungen enthält, einer katalytischen Oligomerisierung (1) unterworfen wird,
- in einer Primärfraktionierung (2) unter Verwendung zumindest eines Teils des Produktgemischs eine Primärfraktion gebildet wird und in einer Sekundärfraktionierung (4) unter Verwendung zumindest eines Teils der Primärfraktion eine Sekundärfraktion gebildet wird,
- die Primärfraktionierung (2) und die Sekundärfraktionierung (4) derart durchgeführt werden, dass die Primärfraktion und die Sekundärfraktion überwiegend eines der Alpha-Olefine enthalten und arm an oder frei von anderen Alpha-Olefinen sind, die Primärfraktion eine oder mehrere der Nebenverbindungen enthält, und die Sekundärfraktion gegenüber der Primärfraktion an der einen oder an den mehreren Nebenverbindungen abgereichert oder frei hiervon ist, und
- in einem Zwischenschritt (3) zwischen der Primärfraktionierung und der Sekundärfraktionierung, welchem zumindest ein Teil der Primärfraktion unterworfen wird, die eine oder die mehreren Nebenverbindungen zumindest zu einem Teil zu einer oder zu mehreren Folgeverbindungen umgesetzt werden und die eine oder die mehreren Folgeverbindungen in der Sekundärfraktionierung zumindest zum Teil abgetrennt werden,
**dadurch gekennzeichnet, dass**
- der Zwischenschritt (3) derart durchgeführt wird, dass nicht mehr als 0,8% des in der Primärfraktion oder deren dem Zwischenschritt unterworfenem Teil überwiegend enthaltenen Alpha-Olefins umgesetzt werden.

2. Verfahren nach Anspruch 1, bei dem der Zwischenschritt in Anwesenheit eines Reaktionsmoderators durchgeführt wird.

3. Verfahren nach Anspruch 2, bei dem als Reaktionsmoderator Wasser in einem Gehalt von 30 Gew.-ppm bis 200 Gew.-ppm verwendet wird.

4. Verfahren nach einem der vorstehenden Ansprüche, bei dem der Zwischenschritt unter Verwendung eines stark sauren Ionenaustauscherharzes durchgeführt wird.

5. Verfahren nach Anspruch 4, bei dem als das stark saure lonenaustauscherharz ein makroporöses Sulfokationit verwendet wird.

6. Verfahren nach Anspruch 4 oder 5, bei dem das stark saure lonenaustauscherharz eine volumenbezogene Kapazität von mindestens 4 eq/kg aufweist.

7. Verfahren nach einem der Ansprüche 4 bis 6, bei dem der Zwischenschritt bei einer Bettgeschwindigkeit von 5 bis 40 h⁻¹ durchgeführt wird.

8. Verfahren nach einem der Ansprüche 3 bis 6, bei dem 0,1 bis 0,8% des in der Primärfraktion oder deren dem Zwischenschritt unterworfenem Teil überwiegend enthaltenen Alpha-Olefins umgesetzt werden.

9. Verfahren nach Anspruch 1, bei dem der Zwischenschritt unter Verwendung eines aluminiumoxidbasierten Katalysators durchgeführt wird.

10. Verfahren nach Anspruch 9, bei dem der aluminiumoxidbasierte Katalysator Chi- und Gamma-Dialuminiumtrioxid aufweist.

11. Verfahren nach Anspruch 9 oder 10, bei dem der aluminiumoxidbasierte Katalysator eine Oberfläche von 450 bis 460 m²/g aufweist.

12. Verfahren nach einem der Ansprüche 9 bis 11, bei dem der Zwischenschritt bei einer Bettgeschwindigkeit von 1 bis 12 h⁻¹ durchgeführt wird.

13. Verfahren nach einem der Ansprüche 9 bis 12, bei dem weniger als 0,2% des in der Primärfraktion oder deren dem Zwischenschritt unterworfenem Teil überwiegend enthaltenen Alpha-Olefins umgesetzt werden.

14. Verfahren nach einem der vorstehenden Ansprüche, bei dem der Zwischenschritt auf einem Temperaturniveau von 60 °C bis 100 °C dur chgeführt wird und/oder auf einem Druckniveau von 1,0 bis 4,0 bar Absolutdruck durchgeführt wird.
